(19) **Europäisches Patentamt / European Patent Office / Office européen des brevets**

(11) **EP 2 932 957 B1**

(12) **EUROPEAN PATENT SPECIFICATION**

(45) Date of publication and mention
of the grant of the patent:
**19.07.2017 Bulletin 2017/29**

(51) Int Cl.:
*A61K 8/36* (2006.01)   *A61K 8/37* (2006.01)
*A61K 8/39* (2006.01)   *A61Q 5/02* (2006.01)
*A61K 8/86* (2006.01)   *A61Q 19/10* (2006.01)

(21) Application number: **15163986.1**

(22) Date of filing: **17.04.2015**

(54) **CLEANSING COSMETICS**

REINIGUNGSKOSMETIKA

PRODUITS COSMÉTIQUES DE NETTOYAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2014 JP 2014085768**

(43) Date of publication of application:
**21.10.2015 Bulletin 2015/43**

(73) Proprietor: **NOF Corporation
20-3 Ebisu 4-chome, Shibuya-ku
Tokyo 150-6019 (JP)**

(72) Inventors:
• **Kochi, Jun-ichi**
**Kawasaki-shi, Kanagawa 2100865 (JP)**
• **Wakita, Kazuaki**
**Kawasaki-shi, Kanagawa 2100865 (JP)**

(74) Representative: **Wills, Andrew Jonathan et al
Mewburn Ellis LLP
City Tower
40 Basinghall Street
London EC2V 5DE (GB)**

(56) References cited:
**JP-A- 2009 249 324     US-A1- 2003 180 335
US-A1- 2005 180 942     US-A1- 2012 164 094**

**Description**

[Technical Field]

The present invention relates to cleansing cosmetics capable of being used to remove oily makeup cosmetics such as foundation, mascara, and lipstick, and capable of being used for body shampoo, hand soap, hair shampoo or the like.

[Background Art]

The primary purpose of cleansing cosmetics is to remove oily makeup cosmetics such as foundation, mascara, and lipstick. Cleansing cosmetics include milky lotion or cream type cosmetics, which demonstrate cleansing properties by the phase reversal of an O/W emulsion with massaging, and oil-type cosmetics where a surfactant is dissolved in oil. Recently, oil-type cleansing cosmetics are generally used since these cosmetics are capable of removing makeups easily and quickly.

However, while such oil-type cleansing cosmetics has excellent cleansing power, these cosmetics have poor slipperiness during cleansing and leave a sticky feeling after washing due to its high oil content. Furthermore, since oil and sebum are highly compatible, the cosmetics remove sebum excessively and thus reduce the moisture-retaining property of skin.

Based on these technical backgrounds, a skin detergent composition containing ester oil such as fatty acid ester and fatty acid triglyceride, a polyoxyethylene polyalcohol fatty acid ester type nonionic surfactant, and fatty acid, and having excellent cleansing power and a good rinsing property, is proposed (PTL 1). However, the skin detergent composition in PTL 1 does not have sufficient cleansing power and moist feeling after cleansing.

Also, a cleansing cosmetic containing water, a polyoxyethylene polyalcohol fatty acid ester type nonionic surfactant, and a higher molecular compound, were proposed (PTL 2). The cleansing cosmetic disclosed in PTL 2 has less irritative property and prevents skin roughening. However, the cleansing cosmetics of PTL 2 have insufficient cleansing power, slipperiness during cleansing, and moist feeling after cleansing.

An oil-type cleansing cosmetic containing a higher fatty acid and a higher alcohol, a liquid oil, a polyhydric alcohol, a polyoxyethylene glyceryl fatty acid ether and a polyoxypropylene diglyceryl ether has been proposed (PTL 3).

A method for producing a high-viscosity o/w cream containing a nonionic surfactant, a linear higher alcohol that has 16 or more carbon atoms and can form an $\alpha$-gel with water, an oil component and water has been proposed (PTL 4).

A skin treatment composition containing an alkylene oxide derivative which has a moisturizing effect, a rough skin improving effect, a stickiness improving effect and a transdermal absorption promoting effect has been proposed (PTL 5). When a refreshing agent is used together with the skin treatment composition of PTL 5, the refreshing effect lasts for a long time and it is excellent in feeling of use without skin stimulation.

A cleansing preparation containing a nonionic surfactant, a liquid oil ingredient and water has been proposed (PTL 6). The cleansing preparation disclosed in PTL 6 takes a clear liquid form, develops neither a reduction in cleansing power nor deteriorations in appearance and sensation upon application even when water is mixed in, and has resistance to water.

[Citation List]

[Patent Literature]

[PTL 1] Japanese Unexamined Patent Application Publication No. 2004-217640
[PTL 2] Japanese Unexamined Patent Application Publication No. 2009-227629
[PTL 3] Japanese Unexamined Patent Application Publication No. 2009-249324
[PTL 4] Japanese Unexamined Patent Application Publication No. 2011-072310
[PTL 5] US Patent Application Publication No. 2003-180335
[PTL 6] US Patent Application Publication No. 2005-180942

[Summary of Invention]

[Technical Problem]

In view of the problems above, an object of the present invention is to provide cleansing cosmetics capable of providing high cleansing power, excellent slipperiness during cleansing, little stickiness after cleansing, and further moist feeling after cleansing.

[Solution to Problem]

After extensive studies to solve the above problems, the inventors have found that it is possible to improve cleansing power, slipperiness during cleansing, sticky feeling after cleansing, and moist feeling after cleansing by using a composition containing the following components (a) to (e) at specific contents respectively, thus completing the present invention.

It is speculated that the components (a), (b) and (d) are densely lined up at an interface between water and an oil component, thus enabling a thick interfacial layer to form to thereby achieve these effects.

Specifically, a cleansing cosmetic of the present invention contains 5 to 20 mass% of the component (a), 10 to 50 mass% of the component (b), 0.5 to 5 mass% of the component (c), 8 to 25 mass% of the component (d), and 25 to 57 mass% of the component (e). It is noted that the total mass of the components (a), (b), (c), (d), and (e) is 100 mass%.

(a) One or more polyalkylene glycol derivatives selected from the group consisting of polyalkylene glycol derivatives represented by Formula (1) and polyalkylene glycol derivatives represented by Formula (2):

$$RO[(EO)_j/(PO)_k]\text{-}H \dots \qquad (1)$$

wherein R is a 4-6C alkyl group; EO is an oxyethylene group; PO is an oxypropylene group; $j$ and $k$ satisfy $5 \leq j \leq 15$ and $5 \leq k \leq 15$ independently; a ratio $[j/k]$ is 0.5 to 2; and EO and PO are randomly added;

$$H(PO)_lO[CH_2CH(O(PO)_mH)CH_2O]_x(PO)_nH \dots \qquad (2)$$

wherein x is $1 \leq x \leq 3$; PO is an oxypropylene group; and $[l + m + n]$ is $5 \leq l + m + n \leq 20$;
(b) one or more ester oils selected from the group consisting of fatty acid esters having a total carbon number of 15 to 30 and fatty acid triglycerides having a total carbon number of 25 to 35, the ester oils being liquid at 25°C;
(c) branched or unsaturated fatty acid having a carbon number of 16 to 18;
(d) a polyoxyethylene polyalcohol fatty acid ester type nonionic surfactant, the carbon number of fatty acid, as a material for the polyalcohol fatty acid ester, is 8 to 22; and
(e) water.

[Advantageous Effects of Invention]

The cleansing cosmetics of the present invention are capable of providing high cleansing power, excellent slipperiness during cleansing, little stickiness after cleansing, and further moist feeling after cleansing.

[Description of Embodiments]

The embodiments of the present invention will be explained below. The cleansing cosmetics of the present invention contains the following components (a), (b), (c), (d) and (e). Hereinafter, each component will be explained.

[Component (a)]

The component (a) for use in the present invention is one or more polyalkylene glycol derivatives selected from the group consisting of polyalkylene glycol derivatives represented by the above-noted Formula (1) and polyalkylene glycol derivatives represented by the above-noted Formula (2).

In Formula (1), R is a 4-6C alkyl group, and may be straight or branched. Specifically, examples of R include an n-butyl group, an isobutyl group, an n-hexyl group, and an isohexyl group. An n-butyl group and an n-hexyl group are preferable, and an n-butyl group is more preferable. When the carbon number of R is less than four or more than six, cleansing power and slipperiness during cleansing may be insufficient.

In Formula (1), EO is an oxyethylene group; PO is an oxypropylene group; and $j$ and $k$ satisfy $5 \leq j \leq 15$ and 5

≤ k ≤ 15 independently.

When j is less than 5 or more than 15, cleansing power and slipperiness during cleansing may be insufficient. j is preferably 6 ≤ j ≤ 14, or more preferably 7 ≤ j ≤ 13.

Similarly, when k is less than 5 or more than 15, cleansing power and slipperiness during cleansing may be insufficient. k is preferably 6 ≤ k ≤ 14, or more preferably 7 ≤ k ≤ 13.

The j in Formula (1) represents an average molar number of ethylene oxide added in Formula (1), and the k in Formula (1) represents an average molar number of propylene oxide added in Formula (1).

The molar ratio [j/k] of average molar number of ethylene oxide added relative to average molar number of propylene oxide added is 0.5 to 2, preferably 0.6 to 1.8, and more preferably 0.7 to 1.6. When the molar ratio [j/k] is less than 0.5 or more than 2, cleansing power and slipperiness during cleansing may be insufficient.

In the polyalkylene glycol derivative of Formula (1), EO and PO are randomly added. When EO and PO are added in blocks, cleansing power and slipperiness during cleansing may be insufficient.

Formula (2) is a polyalkylene glycol derivative prepared by adding propylene oxide to glycerol or polyglycerol.

In Formula (2), x represents the mean degree of polymerization of polyglycerol, and is 1 ≤ x ≤ 3. Examples of polyglycerol include glycerol (x = 1), diglycerol (x = 2), and triglycerol (x = 3). When x exceeds 3, not only may cleansing power and slipperiness during cleansing be insufficient but also a sticky feeling may result after cleansing. x is preferably 1 to 2, or more preferably 2.

In Formula (2), PO is an oxypropylene group; and 1, m and n represent an average molar number of propylene oxide added in Formula (2) respectively. The total of average molar number of propylene oxide added [1 + m + n] is 5 ≤ l + m + n ≤ 20, preferably 6 ≤ l + m + n ≤ 18, and more preferably 7 ≤ l + m + n ≤ 16. When [1 + m + n] is less than 5 or more than 20, cleansing power and slipperiness during cleansing may be insufficient.

Further, in view of cleansing power and slipperiness during cleansing, IOB of the component (a) is preferably 0.80 to 1.30, more preferably 0.85 to 1.20, and still more preferably 0.90 to 1.10. Here, the IOB refers to a ratio between an inorganic value and an organic value (Inorganic Organic Balance) determined on the basis of the Organic Conceptual Diagram (Atsushi Fujita, Prediction of Organic Compounds and Organic Conceptual Diagram, Journal of Japanese Chemistry Vol. 11, No. 10 (1957), 719-725). The IOB is calculated by the following equation.

$$\text{IOB Value} = (\text{Inorganic Value})/(\text{Organic Value})$$

Further, in view of cleansing power and slipperiness during cleansing, the mean molecular weight of the component (a) is preferably 500 to 1500, more preferably 550 to 1300, or still more preferably 600 to 1100. It is noted that the mean molecular weight may be calculated from a method as below using the hydroxyl value described in JIS K-1557 1.

The mean molecular weight of polyalkylene glycol derivatives represented by the Formula (1) = 56110 / hydroxyl value

The mean molecular weight of polyalkylene glycol derivatives represented by the Formula (2) = 168330 / hydroxyl value.

For the component (a), one kind selected from the group consisting of polyalkylene glycol derivatives represented by the Formula (1) and polyalkylene glycol derivatives represented by the Formula (2) may be used, or two or more kinds thereof may be combined. When using the combination of two or more polyalkylene glycol derivatives, the IOB may be calculated as a weighted average of IOB values of the components.

The content of the component (a) is 5 to 20 mass%, preferably 5 to 18 mass%, or more preferably 5 to 15 mass%, based on the total mass of the cleansing cosmetic (100 mass%). When the content of the component (a) is less than 5 mass%, cleansing power and slipperiness during cleansing may be insufficient. When the content is more than 20 mass%, not only may slipperiness during cleansing be insufficient but also stickiness may result after cleansing.

[Component (b)]

The component (b) for use in the present invention is one or more ester oils selected from the group consisting of fatty acid esters having a total carbon number of 15 to 30 and fatty acid triglycerides having a total carbon number of 25 to 35, and the ester oils are liquid at 25°C.

The fatty acid ester of the component (b) has a total of 15 to 30, preferably 17 to 24, or more preferably 24 carbon atoms. When the total carbon number within a molecule is less than 15, cleansing power, slipperiness during cleansing, and moist feeling after cleansing may be insufficient. Also, when the total carbon number is more than 30, not only may cleansing power and slipperiness during cleansing be insufficient but also stickiness may result after cleansing.

The fatty acid triglyceride of the component (b) has a total of 25 to 35, preferably 27 to 33, or more preferably 27 to 30 carbon atoms. When the total carbon number within a molecule is less than 25, cleansing power, slipperiness during cleansing, and moist feeling after cleansing may be insufficient. When the total carbon numbers is more than 35, not only may cleansing power and slipperiness during cleansing be insufficient, but stickiness may result after cleansing.

The fatty acid ester is an ester oil of fatty acid and monovalent alcohol. The fatty acid ester for use in the present invention is liquid at 25°C. Examples of the fatty acid ester specifically include isostearyl pivalate (23C), cetyl 2-ethylhexanoate (24C), isocetyl 2-ethylhexanoate (24C), stearyl 2-ethylhexanoate (26C), isostearyl 2-ethylhexanoate (26C), 2-ethylhexyl isononanoate (17C), isononyl isononanoate (18C), isodecyl isononanoate (19C), isotridecyl isononanoate (22C), isopropyl myristate (17C), isocetyl myristate (30C), isopropyl palmitate (19C), 2-ethylhexyl palmitate (24C), 2-ethylhexyl isopalmitate (24C), isopropyl isostearate (21C), isodecyl isostearate (28C), ethyl oleate (21C), and ethyl linoleate (20C). Cetyl 2-ethylhexanoate (24C), isopropyl myristate (17C), isopropyl palmitate (19C), 2-ethylhexyl palmitate (24C), 2-ethylhexyl isopalmitate (24C), and ethyl oleate (21C) are preferable, or cetyl 2-ethylhexanoate (24C), 2-ethylhexyl palmitate (24C), and 2-ethylhexyl isopalmitate (24C) are more preferable.

Fatty acid triglyceride is a triester oil of fatty acid and glycerol. The fatty acid triglyceride for use in the present invention is liquid at 25°C. Examples of the triglyceride specifically include glyceryl tricaprate (33C), glyceryl tri(2-ethylhexanoate) (27C), glyceryl tricaprylate (27C), and caprylic/capric triglyceride (28C). Glyceryl tricaprate (33C), glyceryl tri(2-ethylhexanoate) (27C), caprylic/capric triglyceride (28C), and glyceryl tri(2-ethylhexanoate) (27C) are preferable, or glyceryl tricaprate (33C) and glyceryl tri(2-ethylhexanoate) (27C) are more preferable.

The content of the component (b) is 10 to 50 mass%, preferably 20 to 45 mass%, more preferably 22 to 40 mass%, or still more preferably 25 to 35 mass%, based on the total mass (100 mass%) of the cleansing cosmetics. When the content of the component (b) is less than 10 mass%, cleansing power, slipperiness during cleansing, and moist feeling after cleansing may be insufficient. When the content exceeds 50 mass%, not only may slipperiness during cleansing be insufficient but also stickiness may result after cleansing.

[Component (c)]

The component (c) for use in the present invention is branched or unsaturated fatty acid having the carbon number of 16 to 18.

The carbon number of the component (c) is 16 to 18, and is preferably 18. When the carbon number is less than 16, cleansing power, slipperiness during cleansing, and moist feeling after cleansing may be insufficient. When the carbon number is more than 18, not only may cleansing power and slipperiness during cleansing be insufficient but also stickiness may result after cleansing.

Examples of the component (c) specifically include palmitoleic acid, isopalmitic acid, oleic acid, isostearic acid, and the like. Isostearic acid and oleic acid are preferable. Among them, one or more fatty acids may be used.

The content of the component (c) is 0.5 to 5 mass%, preferably 0.5 to 4 mass%, or more preferably 0.5 to 3 mass%, based on the total mass (100 mass%) of the cleansing cosmetics. When the content of the component (c) is less than 0.5 mass%, cleansing power, slipperiness during cleansing, and moist feeling after cleansing may be insufficient. When the content is more than 5 mass%, not only may slipperiness during cleansing be insufficient but also stickiness may result after cleansing.

[Component (d)]

The component (d) for use in the present invention is a polyoxyethylene polyalcohol fatty acid ester type nonionic surfactant, and is prepared by adding ethylene oxide to polyalcohol fatty acid ester. Hereinafter, "polyoxyethylene polyalcohol fatty acid ester type nonionic surfactant" may be simply referred to as "nonionic surfactant". Furthermore, "nonionic surfactant", "polyoxyethylene" and "polyoxypropylene" are often referred to as "nonion", "POE" and "POP", respectively.

Examples of polyalcohol, as a material for the polyalcohol fatty acid ester, include glycerol, trimethylolpropane, erythritol, pentaerythritol, sorbitan, diglycerol, xylitol, triglycerol, sorbitol, dipentaerythritol, inositol, and tetraglycerol, and is preferably glycerol, sorbitan, and sorbitol.

The carbon number of fatty acid, as a material for polyalcohol fatty acid ester, is 8 to 22, preferably 10 to 20, or more preferably 12 to 18. Examples of the fatty acid, as a material for polyalcohol fatty acid ester, specifically include caprylic acid, capric acid, undecylenic acid, lauric acid, tridecanoic acid, myristic acid, pentadecylic acid, palmitic acid, margaric acid, stearic acid, arachidic acid, behenic acid, dodecenoic acid, tetradecenoic acid, palmitoleic acid, oleic acid, vaccenic acid, linoleic acid, linolenic acid, arachidonic acid, isooctanoic acid, isononanoic acid, isodecanoic acid, isotridecanoic acid, isotetradecanoic acid, isopalmitic acid, isostearic acid, and hydroxystearic acid. In view of cleansing power and slipperiness during cleansing, lauric acid, oleic acid, and isostearic acid are preferable.

Examples of the nonionic surfactant for the component (d) include POE sorbitan fatty acid ester, POE sorbitol fatty acid ester, POE glycerol fatty acid ester, POE diglycerol fatty acid ester, POE hydrogenated castor oil, POE hydrogenated castor oil fatty acid ester, and POE castor oil. POE glycerol fatty acid ester, POE sorbitan fatty acid ester, and POE sorbitol fatty acid ester are preferable.

By combining a component (d) having low HLB with a component (d) having high HLB, the nonionic surfactant, that is the component (d), is capable of further improving cleansing power and slipperiness during cleansing. Specifically, the HLB range of the component (d) having low HLB is 8.0 to 13.0, preferably 9.0 to 12.0, more preferably 10.0 to 11.5, or still more preferably 10.0 to 11.0. The HLB range of the component (d) having high HLB is 14.0 to 18.0, preferably 14.5 to 17.5, or more preferably 15.0 to 17.0. Here, HLB may be calculated from the following equation (Atlas method).

$$HLB = 20(1 - S/A)$$

S: Saponification value (S.V.) of a nonionic surfactant [JIS K-0070 4.1] A: Acid value (A.V.) of a fatty acid material [JIS K-0070 3.1]

Examples of the component (d) having low HLB specifically include POE(7)glyceryl mono-coconut oil fatty acid (HLB 13.0), POE(5)glyceryl monoisostearate (HLB 10.2), POE(8)glyceryl monoisostearate (HLB 12.0), POE(10)glyceryl monoisostearate (HLB 12.9), POE(20)glyceryl triisostearate (HLB 10.4), POE(30)glyceryl triisostearate (HLB 12.3), POE(6)sorbitan monooleate (HLB 11.8), POE(20)sorbitan trioleate (HLB 10.8), POE(30)sorbitol tetraoleate (HLB 11.2), POE(40)sorbitol tetraoleate (HLB 12.5), POE(30)sorbitol tetraisostearate (HLB 11.1), and POE(40)sorbitol pentaoleate (HLB 11.4). In view of cleansing power and slipperiness during cleansing, POE(20)glyceryl triisostearate (HLB 10.4), POE(20)sorbitan trioleate (HLB 10.8), POE(30)sorbitol tetraoleate (HLB 11.2), POE(30)sorbitol tetraisostearate (HLB 11.1), and POE(40)sorbitol pentaoleate (HLB 11.4) are preferable, and POE(20)glyceryl triisostearate (HLB 10.4) is more preferable.

Examples of the component (d) with high HLB specifically include POE(12)glyceryl monolaurate (HLB 15.0), POE(20)glyceryl monolaurate (HLB 16.5), POE(30)glyceryl monolaurate (HLB 17.5), POE(30)glyceryl mono-coconut oil fatty acid (HLB 17.4), POE(15)glyceryl monoisostearate (HLB 14.4), POE(20)glyceryl monoisostearate (HLB 15.4), POE(30)glyceryl monoisostearate (HLB 16.8), POE(20)glyceryl monooleate (HLB 15.4), POE(30) glyceryl monooleate (HLB 16.6), POE(10)sorbitan monolaurate (HLB 14.9), POE(20)sorbitan monolaurate (HLB 16.7), POE(20)sorbitan monooleate (HLB 15.7), POE(20)sorbitan monoisostearate (HLB 15.7), and POE(60)sorbitol tetraoleate (HLB 14.2). In view of cleansing power and slipperiness during cleansing, POE(20)glyceryl monolaurate (HLB 16.5), POE(20)glyceryl monoisostearate (HLB 15.4), POE(20)glyceryl monooleate (HLB 15.4), POE(20)sorbitan monolaurate (HLB 16.7), POE(20)sorbitan monooleate (HLB 15.7), and POE(20)sorbitan monoisostearate (HLB 15.7) are preferable, and POE(20)glyceryl monolaurate (HLB 16.5), POE(20)glyceryl monoisostearate (HLB 15.4), and POE(20)sorbitan monoisostearate (HLB 15.7) are more preferable.

The content of the component (d) is 8 to 25 mass%, preferably 12 to 24 mass%, or more preferably 16 to 23 mass%, based on the total mass (100 mass%) of the cleansing cosmetics. When the content of the component (d) is less than 8 mass%, cleansing power and slipperiness during cleansing may be insufficient. When the content is more than 25 mass%, not only may slipperiness during cleansing be insufficient but also stickiness may result after cleansing.

[Component (e)]

The component (e) for use in the present invention is water. For example, purified water such as distilled water and deionized water may be preferably used.

The content of the component (e) is 25 to 57 mass%, preferably 30 to 55 mass%, or more preferably 35 to 50 mass%, based on the total mass (100 mass%) of the cleansing cosmetics. When the content of the component (e) is less than 25 mass% or more than 57 mass%, cleansing power and slipperiness during cleansing may be insufficient.

The cleansing cosmetics of the present invention may contain any other components as long as they do not reduce the effect of the invention. For example, it is possible to properly blend the cleansing cosmetics of the invention with oils and fats, hydrocarbon oil, higher alcohols, silicones, a surfactant (however, the component (d) being excluded), a solvent, a pH control agent, an antioxidant, polysaccharide, amino acid, peptide, preservative, fragrance, a coloring agent, and the like.

The cleansing cosmetics of the present invention may be used for body shampoo, hand soap, hair shampoo and the like, besides cleansing cosmetics for removing makeup cosmetics.

[Examples]

The present invention is further described in concrete terms by way of examples and comparative examples. The contents of the components (a), (b), (c), (d), and (e) are expressed in mass% based on the total mass of the components (a), (b), (c), (d), and (e), and the contents of other components are expressed in mass parts when the total mass of the components (a), (b), (c), (d), and (e) is regarded as 100 mass parts.

<Preparation of Cleansing Cosmetics>

Respective Materials of Examples 1 to 10 and Comparative Examples 1 to 7, shown in Table 1 and Table 2, are all placed into a beaker and stirred for one hour with a propeller to prepare cleansing cosmetics.

<Evaluation of Cleansing Cosmetics>

The cleansing cosmetics were tested to evaluate impressions of use by twenty professional panelists. Makeup was removed by using the prepared cleansing cosmetics, then the following four categories were evaluated: (1) cleansing power; (2) slipperiness during cleansing; (3) sticky feeling after cleansing; and (4) moist feeling after cleansing.

(1) Cleansing Power

Makeup was removed with the cleansing cosmetics, then each professional panelist rated its cleansing power by using the following four evaluation levels. Then, on the basis of the total of ratings, the cleansing power was evaluated by using the following five levels, which are "◎◎" to "×". "◎◎" and "◎" were considered as passing. However, when one or more panelists rated a cleansing cosmetic at 0, even if the cleansing cosmetic was evaluated as "◎◎" or "◎", it was considered as failure.

<Evaluation Criteria>

(Rating): (Evaluation)

3: Extremely good in makeup removal.
2: Good in makeup removal.
1: Some makeup were removed.
0: Makeup were hardly removed.

<Five Levels of Evaluation Based on Total of Ratings>

◎◎: total ratings is greater than or equal to 50 points, and less than or equal to 60 points.
◎: total ratings is greater than or equal to 40 points, and less than 50 points.
○: total ratings is greater than or equal to 30 points, and less than 40 points.
Δ: total ratings is greater than or equal to 20 points, and less than 30 points.
×: total ratings is less than 20 points.

(2) Slipperiness during Cleansing

Each professional panelist rated slipperiness during removing makeup with the cleansing cosmetics by using the following four evaluation levels. Then, on the basis of the total of ratings, the slipperiness was evaluated by using the following five levels, which are "◎◎" to "×". "◎◎" and "◎" were considered as passing. However, when one or more panelists rated a cleansing cosmetic at 0, even if the cleansing cosmetic was evaluated as "◎◎" or "◎", it was considered as failure.

<Evaluation Criteria>

(Rating): (Evaluation)

3: Extremely good slipperiness.
2: Moderate slipperiness.

1: Slight slipperiness.
0: Almost no slipperiness.

<Five Levels of Evaluation Based on Total of Ratings>

◎◎: total ratings is greater than or equal to 50 points, and less than or equal to 60 points.
◎: total ratings is greater than or equal to 40 points, and less than 50 points.
○: total ratings is greater than or equal to 30 points, and less than 40 points.
Δ: total ratings is greater than or equal to 20 points, and less than 30 points.
×: total ratings is less than 20 points.

(3) Sticky Feeling after Cleansing

Each professional panelist rated stickiness after removing makeup with the cleansing cosmetics and washing with water by using the following four evaluation levels. Then, on the basis of the total of ratings, the sticky feeling was evaluated by using the following five levels, which are "◎◎" to "×". "◎◎" and "◎" were considered as passing. However, when one or more panelists rated a cleansing cosmetic at 0, even if the cleansing cosmetic was evaluated as "◎◎" or "◎", it was considered as failure.

<Evaluation Criteria>

(Rating): (Evaluation)

3: Stickiness was not felt at all.
2: Stickiness was hardly felt.
1: Slightly sticky.
0: Extremely sticky

<Five Levels of Evaluation Based on Total of Ratings>

◎◎: total ratings is greater than or equal to 50 points, and less than or equal to 60 points.
◎: total ratings is greater than or equal to 40 points, and less than 50 points.
○: total ratings is greater than or equal to 30 points, and less than 40 points.
Δ: total ratings is greater than or equal to 20 points, and less than 30 points.
×: total ratings is less than 20 points.

(4) Moist Feeling after Cleansing

Each professional panelist rated moist feeling after removing makeup with the cleansing cosmetics and washing with water by using the following four evaluation levels. Then, on the basis of the total of ratings, the moist feeling was evaluated by using the following five levels, which are "◎◎" to "×". "◎◎" and "◎" were considered as passing. However, when one or more panelists rated a cleansing cosmetic at 0, even if the cleansing cosmetic was evaluated as "◎◎" or "◎", it was considered as failure.

<Evaluation Criteria>

(Rating): (Evaluation)

3: There was an extremely good moist feeling.
2: There was a moist feeling.

1: There was hardly any moist feeling.

0: There was no moist feeling at all.

<Five Levels of Evaluation Based on Total of Ratings>

◎◎: total ratings is greater than or equal to 50 points, and less than or equal to 60 points.

◎: total ratings is greater than or equal to 40 points, and less than 50 points.

○: total ratings is greater than or equal to 30 points, and less than 40 points.

Δ: total ratings is greater than or equal to 20 points, and less than 30 points.

×: total ratings is less than 20 points.

The above evaluation results are shown in Table 1 and Table 2.

As shown in Table 1, the cleansing cosmetics of Examples 1 to 10 according to the present invention were capable of providing high cleansing power, excellent slipperiness during cleansing, little stickiness after cleansing, and further moist feeling after cleansing.

Additionally, there was no panelist who gave a 0 for the cleansing cosmetics of Examples 1 to 10 in any evaluation categories.

In comparison, significant effects were not found in Comparative Examples 1 to 7.

Specifically, Comparative Example 1 showed poor cleansing power and slipperiness during cleansing, since it contained a component (a'), which had the average molar numbers of ethylene oxide added and propylene oxide added in Formula (1) outside the ranges of the present invention, instead of the component (a) of the present invention.

Since the component (c) of the present invention was not blended for Comparative Example 2 and Comparative Example 3, they showed insufficient cleansing power, slipperiness during cleansing, and moist feeling after cleansing.

Since the component (a) of the present invention was not blended for Comparative Example 4, it showed poor cleansing power and slipperiness during cleansing.

For Comparative Example 5, the component (a) of the present invention was not blended and, moreover, components (b'), (c'), and (d') were blended instead of the components (b), (c), and (d), and thus, cleansing power, slipperiness during cleansing, and moist feeling after cleansing were insufficient.

Since the component (a) and the component (c) of the present invention were not blended for Comparative Example 6, cleansing power, slipperiness during cleansing, and moist feeling after cleansing were insufficient.

Comparative Example 7 showed poor cleansing power, slipperiness during cleansing, sticky feeling after cleansing, and insufficient moist feeling after cleansing, since it contained a component (a'), which had the mean degree of polymerization of polyglycerol and the average molar numbers of propylene oxide added in Formula (2) outside the ranges of the present invention, instead of the component (a) of the present invention, and did not contain the component (c).

Additionally, there was no panelist who gave a 0 for the cleansing cosmetics of Comparative Examples 1 to 6 in any evaluation categories that were evaluated as "◎◎" and "◎".

[Table 1]

| | Components | Examples | | | | | | | | | |
|---|---|---|---|---|---|---|---|---|---|---|---|
| | | 1 | 2 | 3 | 4 | 5 | 6 | 7 | 8 | 9 | 10 |
| (a) | $C_4H_9O[(EO)_9/(PO)_{10}]$-H Mw 1050, IOB 1.03 | 8 | - | - | - | - | 8 | 8 | 8 | 8 | 12 |
| | $C_4H_9O[(EO)_{10}/(PO)_{15}]$-H Mw 1384, IOB 0.93 | - | 8 | - | - | - | - | - | - | - | - |
| | $C_4H_9O[(EO)_{15}/(PO)_{10}]$-H Mw 1314, IOB 1.21 | - | - | 8 | - | - | - | - | - | - | - |
| | PPG(9)diglycerol Mw 668, IOB 1.05 | - | - | - | 8 | - | - | - | - | - | - |
| | PPG(8)glycerol Mw 556, IOB 1.00 | - | - | - | - | 8 | - | - | - | - | - |
| (b) | 2-ethylhexyl palmitate Carbon number 24 | 25 | 25 | 25 | 25 | 25 | - | - | 25 | 25 | 30 |
| | Isopropyl myristate Carbon number 17 | - | - | - | - | - | 25 | - | - | - | - |
| | Glyceryl tri(2-ethylhexanoate) Carbon number 27 | - | - | - | - | - | - | 25 | - | - | - |
| (c) | Isostearic acid Carbon number 18 | 3 | 3 | 3 | 3 | 3 | 3 | 3 | - | 3 | 1 |
| | Oleic acid Carbon number 18 | - | - | - | - | - | - | - | 3 | - | - |
| (d) | POE(20)glyceryl triisostearate HLB: 10.4 | 15 | 15 | 15 | 15 | 15 | 15 | 15 | 15 | 15 | 13 |
| | POE(20)glyceryl monoisostearate HLB: 15.4 | 5 | 5 | 5 | 5 | 5 | 5 | 5 | 5 | - | - |
| | POE(20)sorbitan monolaurate HLB: 16.7 | - | - | - | - | - | - | - | - | 5 | 2 |
| (e) | Purified water | 44 | 44 | 44 | 44 | 44 | 44 | 44 | 44 | 44 | 42 |
| | Subtotal | 100 | 100 | 100 | 100 | 100 | 100 | 100 | 100 | 100 | 100 |
| Others | Cocamide DEA | 2 | 2 | 2 | 2 | 2 | 2 | 2 | 2 | 2 | 2 |
| | Glycerol | 10 | 10 | 10 | 10 | 10 | 10 | 10 | 10 | 10 | 10 |
| | Total | 112 | 112 | 112 | 112 | 112 | 112 | 112 | 112 | 112 | 112 |
| Evaluation | Cleansing power | ◎◎ | ◎ | ◎ | ◎◎ | ◎ | ◎ | ◎◎ | ◎◎ | ◎◎ | ◎◎ |
| | Slipperiness during cleansing | ◎◎ | ◎ | ◎ | ◎◎ | ◎ | ◎ | ◎◎ | ◎◎ | ◎◎ | ◎◎ |
| | Sticky feeling after cleansing | ◎◎ | ◎◎ | ◎◎ | ◎◎ | ◎◎ | ◎◎ | ◎◎ | ◎◎ | ◎◎ | ◎◎ |
| | Moist feeling after cleansing | ◎◎ | ◎◎ | ◎◎ | ◎◎ | ◎◎ | ◎ | ◎◎ | ◎◎ | ◎◎ | ◎◎ |

[Table 2]

| | Components | Comparative Examples | | | | | | |
|---|---|---|---|---|---|---|---|---|
| | | 1 | 2 | 3 | 4 | 5 | 6 | 7 |
| (a) | $C_4H_9O[(EO)_9/(PO)_{10}]$-H Mw 1050, IOB 1.03 | - | 8 | 8 | - | - | - | - |
| (a') | $C_4H_9O[(EO)_{17}/(PO)_{17}]$-H Mw 1808, IOB 1.07 | 8 | - | - | - | - | - | - |
| | PPG(70)decaglyceryl ether Mw 4818, IOB 0.93 | - | - | - | - | - | - | 8 |
| (b) | 2-ethylhexyl palmitate | 25 | 25 | - | - | - | 25 | 25 |
| | Isopropyl myristate | - | - | 25 | - | - | - | - |
| | Caprylic/capric triglyceride | - | - | - | 25 | - | - | - |
| (b') | Hydrogenated polyisobutene (degree of polymerization: 5) | - | - | - | - | 13 | - | - |
| (c) | Isostearic acid | 3 | - | - | 3 | - | - | - |
| (c') | Lauric acid | - | - | - | - | 8 | - | - |
| (d) | POE(20)glyceryl triisostearate HLB: 10.4 | 15 | 15 | 15 | 15 | - | 15 | 15 |
| | POE(20)glyceryl monoisostearate HLB: 15.4 | 5 | 5 | 5 | 5 | - | 5 | 5 |
| (d') | Polyethylene glycol monolaurate HLB: 14.0 | - | - | - | - | 18 | - | - |
| (e) | Purified water | 44 | 47 | 47 | 52 | 61 | 55 | 47 |
| Subtotal | | 100 | 100 | 100 | 100 | 100 | 100 | 100 |
| Others | Cocamide DEA | 2 | 2 | 2 | 2 | - | 2 | 2 |
| | Glycerol | 10 | 10 | 10 | 10 | - | 10 | 10 |
| | Ethanol | - | - | - | - | 33 | - | - |
| Total | | 112 | 112 | 112 | 112 | 133 | 112 | 112 |
| Evaluation | Cleansing power | × | ○ | Δ | × | ○ | × | × |
| | Slipperiness during cleansing | × | ○ | Δ | × | ○ | × | × |
| | Sticky feeling after cleansing | ◎◎ | ◎◎ | ◎◎ | ◎◎ | ◎◎ | ◎◎ | × |
| | Moist feeling after cleansing | ◎◎ | ○ | Δ | ◎◎ | ○ | Δ | Δ |

Examples 11, 12, and 13 are shown below as reference formulations for the cleansing cosmetics of the present invention. In any of the formulations, the cleansing cosmetics were capable of providing high cleansing power, excellent slipperiness during cleansing, little stickiness after cleansing, and further moist feeling after cleansing.

(Example 11)

Component name (Blending ratio: Mass%)

(a) $C_4H_9O[(EO)_9/(PO)_{10}]$-H (8.0)
(b) 2-ethylhexyl palmitate (15.0)
(b) Isopropyl palmitate (5.0)
(b) Ethyl oleate (5.0)
(c) Isopalmitic acid (3.0)
(d) POE(20)glyceryl triisostearate (15.0)
(d) POE(20)glyceryl monoisostearate (5.0)
(e) Deionized water (44.0)

Subtotal: 100 mass%

(Other Components)

1, 3-butylene glycol (5.0)
Propylene glycol (5.0)
Ethanol (1.0)
Polyethylene glycol 1000 (0.5)
Polyethylene glycol 1540 (0.5)
Sodium citrate (0.5)
Methylparaben (0.1)
Fragrance (0.1)
Dipotassium glycyrrhizate (0.1)
Citric acid (0.1)
Tetrasodium etidronate (0.1)
Xanthan gum (0.05)
(acrylate/alkyl (C10-30) acrylate)crosspolymer (0.05)
Polyquaternium-51 (0.05)
Carbomer (0.05)
Tocopherol (0.01)
Total: 113.21 mass%

(Example 12)

Component name (Blending ratio: Mass%)

(a) $C_4H_9O[(EO)_7/(PO)_{13}]$-H (9.0)
(b) Cetyl 2-ethylhexanoate (15.0)
(b) 2-ethylhexyl isopalmitate (5.0)
(b) Isopropyl myristate (5.0)
(c) Palmitoleic acid (2.0)
(d) POE(20)glyceryl triisostearate (15.0)
(d) POE(20)sorbitan monolaurate (5.0)
(e) Deionized water (44.0)

Subtotal: 100 mass%

(Other Components)

1, 3-butylene glycol (4.0)
Dipropylene glycol (2.0)
Ethanol (2.0)
Polyethylene glycol 1000 (1.0)
Polyethylene glycol 1540 (1.0)
Sodium citrate (0.5)
Phenoxyethanol (0.5)
Fragrance (0.1)
Disodium EDTA (0.1)
Sodium hyaluronate (0.05)
(glycerylamidoethyl methacrylate/stearyl methacrylate)copolymer (0.05)
Tocopherol acetate (0.01)
Ascorbic acid derivative (0.01)
Total: 111.32 mass%

(Example 13)

Component name (Blending ratio: Mass%)

(a) $C_4H_9O[(EO)_5/(PO)_5]$-H (3.0)
(a) $C_4H_9O[(EO)_9/(PO)_{10}]$-H (4.0)
(a) $C_4H_9O[(EO)_{10}/(PO)_7]$-H (3.0)
(a) $C_4H_9O[(EO)_{12}/(PO)_{12}]$-H (3.0)
(b) Ethylhexyl isopalmitate (19.5)
(c) Isostearic acid (2.5)
(d) Sorbeth-30 tetraisostearate (20.5)
(d) Polysorbate 20 (2.5)
(e) Deionized water (42.0)

Subtotal: 100 mass%

(Other Components)

Hydrogenated polyisobutene (6.5)
Cyclopentasiloxane (6.5)
Glycerol (6.5)
Propanediol (6.5)
Polyquaternium-51 (0.1)
Citric acid (0.4)
Cocamide DEA (2.5)
Lauroyl methyl alanine Na (0.8)
Total: 129.8 mass%

**Claims**

1. A cleansing cosmetic containing 5 to 20 mass% of component (a), 10 to 50 mass% of component (b), 0.5 to 5 mass% of component (c), 8 to 25 mass% of component (d), and 25 to 57 mass% of component (e).

   (a) One or more polyalkylene glycol derivatives selected from the group consisting of polyalkylene glycol derivatives represented by Formula (1) and polyalkylene glycol derivatives represented by Formula (2):

   $$RO[(EO)_j/(PO)_k]\text{-}H \ ... \qquad (1)$$

   wherein R is a 4-6C alkyl group; EO is an oxyethylene group; PO is an oxypropylene group; j and k satisfy $5 \leq j \leq 15$ and $5 \leq k \leq 15$ independently; a ratio [j/k] is 0.5 to 2; and EO and PO are randomly added;

   $$H(PO)_lO[CH_2CH(O(PO)_mH)CH_2O]_x(PO)_nH \ ... \qquad (2)$$

   wherein x is $1 \leq x \leq 3$; PO is an oxypropylene group; and [l + m + n] is $5 \leq l + m + n \leq 20$;
   (b) one or more ester oils selected from the group consisting of fatty acid esters having a total carbon number of 15 to 30 and fatty acid triglycerides having a total carbon number of 25 to 35, the ester oils being liquid at 25°C;
   (c) branched or unsaturated fatty acid having a carbon number of 16 to 18;
   (d) a polyoxyethylene polyalcohol fatty acid ester type nonionic surfactant, the carbon number of fatty acid, as material for the polyalcohol fatty acid ester, is 8 to 22; and
   (e) water.

2. The cleansing cosmetic of claim 1, containing:

5 to 18 mass% of component (a), such as 5 to 15 mass%; and/or
20 to 45 mass% of component (b), such as 22 to 40 mass%, such as 25 to 35 mass%; and/or
0.5 to 4 mass% of component (c), such as 0.5 to 3 mass%; and/or
12 to 24 mass% of component (d), such as 16 to 23 mass%; and/or
30 to 55 mass% of component (e), such as 35 to 50 mass%.

3. The cleansing cosmetic of claim 1 or claim 2, wherein:

(i) J is $6 \leq j \leq 14$, such as $7 \leq j \leq 13$; and/or
(ii) k is $6 \leq k \leq 14$, such as $7 \leq k \leq 13$; and/or
(iii) the ratio [j/k] is 0.6 to 1.8, such as 0.7 to 1.6; and/or
(iv) x is 1 or 2, such as 2; and/or
(v) [l + m + n] is $6 \leq l + m + n \leq 18$, such as $7 \leq l + m + n \leq 16$.

4. The cleansing cosmetic of any one of the preceding claims, wherein the IOB of component (a) is 0.80 to 1.30, such as 0.85 to 1.20.

5. The cleansing cosmetic of any one of the preceding claims, wherein the mean molecular weight of the component (a) is 500 to 1500, such as 550 to 1300, such as 600 to 1100.

6. The cleansing cosmetic of any one of the preceding claims, wherein the fatty acid ester of the component (b) has a total of 17 to 24 carbon atoms, such as 24 carbon atoms; and/or Fatty acid triglyceride of the component (b) has a total of 27 to 33 carbon atoms, such as 27 to 30 carbon atoms.

7. The cleansing cosmetic of any one of the preceding claims, wherein:

the fatty acid ester is selected from the group consisting of isostearyl pivalate (23C), cetyl 2-ethylhexanoate (24C), isocetyl 2-ethylhexanoate (24C), stearyl 2-ethylhexanoate (26C), isostearyl 2-ethylhexanoate (26C), 2-ethylhexyl isononanoate (17C), isononyl isononanoate (18C), isodecyl isononanoate (19C), isotridecyl isononanoate (22C), isopropyl myristate (17C), isocetyl myristate (30C), isopropyl palmitate (19C), 2-ethylhexyl palmitate (24C), 2-ethylhexyl isopalmitate (24C), isopropyl isostearate (21C), isodecyl isostearate (28C), ethyl oleate (21C), and ethyl linoleate (20C); and/or
the fatty acid triglyceride is selected from the group consisting of glyceryl tricaprate (33C), glyceryl tri(2-ethylhexanoate) (27C), glyceryl tricaprylate (27C), and caprylic/capric triglyceride (28C).

8. The cleansing cosmetic of any one of the preceding claims, wherein the carbon number of component (c) is 18.

9. The cleansing cosmetic of any one of the preceding claims, wherein the component (c) is one or more fatty acids selected from the group consisting of palmitoleic acid, isopalmitic acid, oleic acid, and isostearic acid.

10. The cleansing cosmetic of any one of the preceding claims, wherein the component (d) comprises one or more polyoxyethylene polyalcohol fatty acid ester type nonionic surfactants selected from the group consisting of POE sorbitan fatty acid ester, POE sorbitol fatty acid ester, POE glycerol fatty acid ester, POE diglycerol fatty acid ester, POE hydrogenated castor oil, POE hydrogenated castor oil fatty acid ester, and POE castor oil.

11. The cleansing cosmetic of any one of the preceding claims, wherein the component (d) comprises a component having a HLB value in the range 8.0 to 13.0, such as 9.0 to 12.0, and a component having a HLB value in the range 14.0 to 18.0, such as 14.5 to 17.5.

12. Use of the cleansing cosmetic of any one of claims 1 to 11 as a cleanser, such as a cleanser for the removal of makeup cosmetics, body shampoo, hand soap, or hair shampoo.

**Patentansprüche**

1. Reinigungskosmetikprodukt, das 5 bis 20 Massen-% von Komponente (a), 10 bis 50 Massen-% von Komponente (b), 0,5 bis 5 Massen-% von Komponente (c), 8 bis 25 Massen-% von Komponente (d) und 27 bis 75 Massen-% von Komponente (e) enthält:

(a) ein oder mehrere Polyalkylenglykol-Derivate, die aus der Gruppe bestehend aus Polyalkylenglykol-Derivaten, die durch Formel (1) dargestellt sind, und aus Polyalkylenglykol-Derivaten, die durch Formel (2) dargestellt sind, ausgewählt sind:

$$RO[(EO)_j/(PO)_k]\text{-}H \dots \qquad (1)$$

worin R eine $C_{4-6}$-Alkylgruppe ist; EO eine Oxyethylengruppe ist; PO eine Oxypropylengruppe ist; j und k jeweils unabhängig $5 \leq j \leq 15$ und $5 \leq k \leq 15$ erfüllen; das Verhältnis [j/k] 0,5:2 beträgt; und EO und PO statistisch addiert sind;

$$H(PO)_lO[CH_2CH(O(PO)_mH)CH_2O]_x(PO)_nH \dots \qquad (2)$$

worin für x gilt: $1 \leq x \leq 3$; PO eine Oxypropylengruppe ist; und für [l + m + n] gilt: $5 \leq l + m + n \leq 20$;

(b) ein oder mehrere Esteröle, die aus der Gruppe bestehend aus Fettsäureestern mit einer Kohlenstoffgesamtanzahl von 15 bis 30 und Fettsäureglyceriden mit einer Kohlenstoffgesamtanzahl von 25 bis 35 ausgewählt sind, wobei die Esteröle bei 25 °C flüssig sind;

(c) verzweigte oder unverzweigte Fettsäure mit einer Kohlenstoffanzahl von 16 bis 18;

(d) ein nichtionisches Tensid vom Polyoxyethylen-Polyalkohol-Fettsäureester-Typ, wobei die Kohlenstoffanzahl der Fettsäure als Material für den Polyalkoholfettsäureester 8 bis 22 beträgt; und

(e) Wasser.

2. Reinigungskosmetikprodukt nach Anspruch 1, das Folgendes enthält:

5 bis 18 Massen-% von Komponente (a), wie z.B. 5 bis 15 Massen-%; und/oder

20 bis 45 Massen-% von Komponente (b), wie z.B. 22 bis 40 Massen-%, wie z.B. 25 bis 35 Massen-%; und/oder

0,5 bis 4 Massen-% von Komponente (c), wie z.B. 0,5 bis 3 Massen-%; und/oder

12 bis 24 Massen-% von Komponente (d), wie z.B. 16 bis 23 Massen-%; und/oder

30 bis 55 Massen-% von Komponente (e), wie z.B. 35 bis 50 Massen-%.

3. Reinigungskosmetikprodukt nach Anspruch 1 oder Anspruch 2, wobei:

(i) für j gilt: $6 \leq j \leq 14$, wie z.B. $7 \leq j \leq 13$; und/oder

(ii) für k gilt: $6 \leq k \leq 14$, wie z.B. $7 \leq k \leq 13$; und/oder

(iii) das Verhältnis [j/k] 0,6 bis 1,8 beträgt, wie z.B. 0,7 bis 1,6; und/oder

(iv) x = 1 oder 2 ist, wie z.B. 2; und/oder

(v) für [l + m + n] gilt: $6 \leq l + m + n \leq 18$, wie z.B. $7 \leq l + m + n \leq 16$.

4. Reinigungskosmetikprodukt nach einem der vorangegangenen Ansprüche, wobei der IOB-Wert von Komponente (a) 0,80 bis 1,30 beträgt, wie z.B. 0,85 bis 1,20.

5. Reinigungskosmetikprodukt nach einem der vorangegangenen Ansprüche, wobei das mittlere Molekulargewicht der Komponente (a) 500 bis 1500 beträgt, wie z.B. 550 bis 1300, wie z.B. 600 bis 1100.

6. Reinigungskosmetikprodukt nach einem der vorangegangenen Ansprüche, wobei der Fettsäureester der Komponente (b) insgesamt 17 bis 24 Kohlenstoffatome, wie z.B. 24 Kohlenstoffatome, aufweist; und/oder das Fettsäuretriglycerid der Komponente (b) insgesamt 27 bis 33 Kohlenstoffatome, wie z.B. 27 bis 30 Kohlenstoffatome, aufweist.

7. Reinigungskosmetikprodukt nach einem der vorangegangenen Ansprüche, wobei:

der Fettsäureester aus der Gruppe bestehend aus Isostearylpivalat (23C), Cetyl-2-ethylhexanoat (24C), Isocetyl-2-ethylhexanoat (24C), Stearyl-2-ethylhexano-at (26C), Isostearyl-2-ethylhexanoat (26C), 2-Ethylhexylisononanoat (17C), Isononyl-isononanoat (18C), Isodecylisononanoat (19C), Isotridecylisononanoat (22C), Isopropylmyristat (17C), Isocetylmyristat (30C), Isopropylpalmitat (19C), 2-Ethylhexylpalmitat (24C), 2-Ethylhexylisopalmitat (24C), Isopropylisostearat (21C), Isodecyl-isostearat (28C), Ethyloleat (21 C) und Ethyllinoleat (20C) ausgewählt ist; und/oder

das Fettsäuretriglycerid aus der Gruppe bestehend aus Glycerintricaprat (33C), Glycerintri(2-ethylhexanoat) (27C), Glycerintricaprylat (27C) und Caprylsäure-/ Caprinsäure-triglycerid (28C) ausgewählt ist.

**8.** Reinigungskosmetikprodukt nach einem der vorangegangenen Ansprüche, wobei die Kohlenstoffanzahl von Komponente (c) 18 beträgt.

**9.** Reinigungskosmetikprodukt nach einem der vorangegangenen Ansprüche, wobei die Komponente (c) eine oder mehrere Fettsäuren ist, die aus der Gruppe bestehend aus Palmitoleinsäure, Isopalmitinsäure, Oleinsäure und Isostearinsäure ausgewählt ist.

**10.** Reinigungskosmetikprodukt nach einem der vorangegangenen Ansprüche, wobei die Komponente (d) ein oder mehr nichtionische Tenside vom Polyoxyethylen-Polyalkoholfettsäureester-Typ umfasst, die aus der Gruppe bestehend aus POE-Sorbitanfettsäureester, POE-Sorbit-Fettsäureester, hydrogeniertem POE-Kastoröl, hydriertem POE-Kastoröl-Fettsäureester und POE-Kastoröl ausgewählt sind.

**11.** Reinigungskosmetikprodukt nach einem der vorangegangenen Ansprüche, wobei die Komponente (d) eine Komponente mit einem HLB-Wert im Bereich von 8,0 bis 13,0, wie z.B. 9,0 bis 12,0, und eine Komponente mit einem HLB-Wert im Bereich von 14,0 bis 18,0, wie z.B. 14,5 bis 17,5, umfasst.

**12.** Verwendung eines Reinigungskosmetikprodukts nach einem der Ansprüche 1 bis 11 als Reinigungsprodukt, wie z.B. als Reinigungsprodukt zum Entfernen von Make-up-Kosmetika, als Körpershampoo, als Handseife oder als Haarshampoo.

**Revendications**

**1.** Produit cosmétique nettoyant contenant de 5 à 20 % en masse de composant (a), de 10 à 50 % en masse de composant (b), de 0,5 à 5 % en masse de composant (c), de 8 à 25 % en masse de composant (d) et de 25 à 57 % en masse de composant (e),

(a) un ou plusieurs dérivés de polyalkylène glycol choisis dans le groupe constitué par les dérivés de polyalkylène glycol représentés par la formule (1) et les dérivés de polyalkylène glycol représentés par la formule (2) :

$$RO[(EO)_j/(PO)_k]\text{-}H \qquad (1)$$

dans laquelle R est un groupe alkyle en $C_4$ à $C_6$ ; EO est un groupe oxyéthylène ; PO est un groupe oxypropylène ; j et k satisfont à $5 \leq j \leq 15$ et $5 \leq k \leq 15$ indépendamment ; un rapport [j/k] est de 0,5 à 2 ; et EO et PO sont aléatoirement ajoutés ;

$$H(PO)_lO[CH_2CH(O(PO)_mH)CH_2O]_x(PO)_nH \qquad (2)$$

dans laquelle x est $1 \leq x \leq 3$ ; PO est un groupe oxypropylène ; et [l + m + n] est $5 \leq l + m + n \leq 20$ ;
(b) une ou plusieurs huiles d'ester choisies dans le groupe constitué par les esters d'acide gras ayant un nombre d'atomes de carbone total de 15 à 30 et les triglycérides d'acide gras ayant un nombre d'atomes de carbone total de 25 à 35, les huiles d'ester étant liquides à 25 °C ;
(c) un acide gras ramifié ou insaturé ayant un nombre d'atomes de carbone de 16 à 18 ;
(d) un tensioactif non ionique de type ester d'acide gras de polyoxyéthylène polyalcool, le nombre d'atomes de carbone de l'acide gras, en tant que matière pour l'ester d'acide gras de polyalcool est de 8 à 22 ; et
(e) de l'eau.

**2.** Produit cosmétique nettoyant selon la revendication 1, contenant :

de 5 à 18 % en masse de composant (a), tel que de 5 à 15 % en masse ; et/ou
de 20 à 45 % en masse de composant (b), tel que de 22 à 40 % en masse, tel que de 25 à 35 % en masse ; et/ou
de 0,5 à 4 % en masse de composant (c), tel que de 0,5 à 3 % en masse ; et/ou
de 12 à 24 % en masse de composant (d), tel que de 16 à 23 % en masse ; et/ou
de 30 à 55 % en masse de composant (e), tel que de 35 à 50 % en masse.

**3.** Produit cosmétique nettoyant selon la revendication 1 ou la revendication 2, dans laquelle :

(i) j est $6 \leq j \leq 14$, tel que $7 \leq j \leq 13$ ; et/ou

(ii) k est 6 ≤ k ≤ 14, tel que 7 ≤ k ≤ 13 ; et/ou
(iii) le rapport [j/k] est de 0,6 à 1,8, tel que de 0,7 à 1,6 ; et/ou
(iv) x vaut 1 ou 2, tel que 2 ; et/ou
(v) [l + m + n] est 6 ≤ l + m + n ≤ 18, tel que 7 ≤ l + m + n ≤ 16.

4. Produit cosmétique nettoyant selon l'une quelconque des revendications précédentes, dans lequel l'IOB du composant (a) est de 0,80 à 1,30, tel que de 0,85 à 1,20.

5. Produit cosmétique nettoyant selon l'une quelconque des revendications précédentes, dans lequel le poids moléculaire moyen du composant (a) est de 500 à 1500, tel que de 550 à 1300, tel que de 600 à 1100.

6. Produit cosmétique nettoyant selon l'une quelconque des revendications précédentes, dans lequel l'ester d'acide gras du composant (b) a un total de 17 à 24 atomes de carbone, tel que de 24 atomes de carbone ; et le triglycéride d'acide gras du composant (b) a un total de 27 à 33 atomes de carbone, tel que de 27 à 30 atomes de carbone.

7. Produit cosmétique nettoyant selon l'une quelconque des revendications précédentes, dans lequel :

l'ester d'acide gras est choisi dans le groupe constitué par le pivalate d'isostéaryle ($C_{23}$), le 2-éthylhexanoate de cétyle ($C_{24}$), le 2-éthylhexanoate d'isocétyle ($C_{24}$), le 2-éthylhexanoate de stéaryle ($C_{26}$), le 2-éthylhexanoate d'isostéaryle ($C_{26}$), l'isononanoate de 2-éthylhexyle ($C_{17}$), l'isononanoate d'isononyle ($C_{18}$), l'isononanoate d'isodécyle ($C_{19}$), l'isononanoate d'isotridécyle ($C_{22}$), le myristate d'isopropyle ($C_{17}$), le myristate d'isocétyle ($C_{30}$), le palmitate d'isopropyle ($C_{19}$), le palmitate de 2-éthylhexyle ($C_{24}$), l'isopalmitate de 2-éthylhexyle ($C_{24}$), l'isostéarate d'isopropyle ($C_{21}$), l'isostéarate d'isodécyle ($C_{28}$), l'oléate d'éthyle ($C_{21}$) et le linoléate d'éthyle ($C_{20}$) ; et/ou

le triglycéride d'acide gras est choisi dans le groupe constitué par le tricaprate de glycéryle ($C_{33}$), le tri(2-éthylhexanoate) de glycéryle ($C_{27}$), le tricaprylate de glycéryle ($C_{27}$) et le triglycéride caprylique/caprique ($C_{28}$).

8. Produit cosmétique nettoyant selon l'une quelconque des revendications précédentes, dans lequel le nombre d'atomes de carbone du composant (c) est 18.

9. Produit cosmétique nettoyant selon l'une quelconque des revendications précédentes, dans lequel le composant (c) est un ou plusieurs acides gras choisis dans le groupe constitué par l'acide palmitoléique, l'acide isopalmitique, l'acide oléique et l'acide isostéarique.

10. Produit cosmétique nettoyant selon l'une quelconque des revendications précédentes, dans lequel le composant (d) comprend un ou plusieurs tensioactifs non ioniques de type ester d'acide gras de polyoxyéthylène polyalcool choisis dans le groupe constitué par un ester d'acide gras de POE sorbitane, un ester d'acide gras de POE sorbitol, un ester d'acide gras de POE glycérol, un ester d'acide gras de POE diglycérol, une huile de ricin hydrogénée de POE, un ester d'acide gras d'huile de ricin hydrogénée de POE et une huile de ricin de POE.

11. Produit cosmétique nettoyant selon l'une quelconque des revendications précédentes, dans lequel le composant (d) comprend un composant ayant une valeur HLB comprise dans la plage allant de 8,0 à 13,0, telle que de 9,0 à 12,0 et un composant ayant une valeur HLB comprise dans la plage allant de 14,0 à 18,0, telle que de 14,5 à 17,5.

12. Utilisation du produit cosmétique nettoyant selon l'une quelconque des revendications 1 à 11 en tant que nettoyant, tel qu'un nettoyant pour l'élimination de produits cosmétiques de maquillage, un shampoing pour le corps, un savon pour les mains ou un shampoing pour les cheveux.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004217640 A
- JP 2009227629 A
- JP 2009249324 A
- JP 2011072310 A
- US 2003180335 A
- US 2005180942 A

**Non-patent literature cited in the description**

- **ATSUSHI FUJITA.** Prediction of Organic Compounds and Organic Conceptual Diagram. *Journal of Japanese Chemistry,* 1957, vol. 11 (10), 719-725